# EUROPEAN PATENT APPLICATION

(11) **EP 2 708 213 A1**
(43) Date of publication of application: **19.03.2014**
(21) Application number: 12184225.6
(22) Date of filing: 13.09.2012
(51) Int. Cl.: A61F 6/14, A61K 9/00

(54) **Multipurpose ethylene vinyl acetate fibrous drug delivery systems for long-term implantation or insertion**

(71) Applicant: PAT&Co bvba, 8810 Lichtervelde (BE)
(72) Inventor: Wildemeersch, Dirk, 9000 Ghent (BE)
(74) Representative: Colens, Alain M.G.M.

(57) **Abstract**

A multipurpose, multicomponent or multisegmented drug intrauterine T-shaped delivery system or device, of which both the stem and the transverse arm of the device are drug eluting compartments which are active in the uterus and/or the cervical canal. The drugs may be different and/or have different concentration and releasing characteristics. The stem is preferably provided with a dome-shaped extremity.

The stem and/or the transverse arm consist of ethylene vinyl acetate which contains a level of vinyl acetate monomer lower than 0.5% w/w.

## Description

The invention relates to new and improved, multipurpose "frameless" and "framed" intrauterine drug delivery systems specifically for contraception and the prevention and treatment of gynecological conditions, including the treatment of deep-seated and voluminous lesion in the genital tract of women, such as fibromyomas and endometriotic/adenomyotic lesions, releasing one or more drugs simultaneously.

Steroids have been used for incorporation into drug delivery systems produced by melt-spinning processes. Data from the applicant have shown that potent progestogens such as levonorgestrel can be processed for long-term release in the genital tract or subdermally. It has also been shown that sustained drug delivery systems can be processed using compounds that antagonize the action of natural progesterone. These systems are highly interesting for the release of these agonist and antagonist agents in the genital tract (e.g., uterus, vagina) using an intrauterine system (IUS) and/or a vaginal ring system (IVR). These systems could also be used for subdermal delivery of the active compound(s).

Since uterine pathologies are often severe, and the lesions to be treated sometimes deep-seated in the uterus or beyond the uterus, a high dose may be required to obtain a therapeutic effect. The way how this can be realized is described in this patent application.

The invention will further disclose IUSs, IVRs and subdermal implant systems that can be used to meet the objective of this application, including the proper choice of the polymer(s) as well as the characteristics of the drug delivery systems to obtain an optimal release profile for the treatment of the above mentioned conditions in women.

### FIELD AND BACKGROUND OF THE INVENTION

Synthetic fibers can be used for nonmedical and medical applications. In the medical field, fibers can be used as controlled-delivery systems such that, when bioactive agents are combined with synthetic polymers, and extruded to form filaments, precision drug delivery systems can be manufactured in a continuous process. These fibers can be prepared using various techniques including high speed extrusion systems. Moreover, the versatility, speed, and economy of the fiber-spinning processes make the production of controlled-release fibrous systems attractive for use in the medical field as cost is at present a major factor in marketing a controlled-release product.

As with other types of controlled-release systems, fibers can be designed to release various bioactive materials by a number of different mechanisms including diffusion, dissolution, erosion, and biodegradation. The choice of polymer(s) used, and the rate of release, will depend upon the particular bioactive agent and the fibrous delivery system. Both first-order and zero-order (constant) rates of release can be obtained, with some systems giving release rates that appear to be a combination of the two. Thus, complex pseudo constant rates of release may be obtained from some fibrous devices.

Two approaches can be used for designing fibers that exhibit zero-order or apparent zero-order release of agent over prolonged period of time. In one approach, the agent is dissolved or homogenously dispersed within the polymer matrix, and subsequently the fiber is coated with a membrane of the same or a second polymer that controls the release rate. Alternatively, hollow or coaxial fibers containing the bioactive agent within the core are prepared. In the latter case, constant release of the agent is achieved without further modification of the fiber since the wall of the coaxial fiber serves as the rate-controlling membrane.

Coaxial spinning, rather than filling of spun hollow fibers, is most practical and economical. Special equipment is necessary to make coaxial fibers as both inner core and outer rate-controlling membrane are extruded at the same time. This procedure gives a quick and reliable method for producing a reservoir system with a known amount of active agent.

In our laboratories, we have prepared both monolithic and reservoir devices from fibers, as well as some that are hybrids of the two, using melt-spinning processes. These include fibers that are coated on all surfaces except for the proximal and distal ends, fibers that are closed at the ends, fibers that have holes, fibers that have different geometries, and fibers with different ratios of surface area to volume. All of these variations have been used to control the rate of release, the quantity of the agent released, and the duration of release. Representative examples of fibrous delivery systems were developed with different kinds of bioactive substances such as, contraceptive steroids, antimicrobial agents and antibiotics.

The area of contraception has greatly benefited from the advances made in controlled drug delivery. The most successful controlled drug delivery system to date is the family of implantable female contraceptive devices developed by the Population Council in the mid-1970s. Norplant, the first of a series of implantable contraceptive delivery devices, is capable of delivering levonorgestrel (LNG) at efficacious levels from the time of implantation up to a period of 5 years. This has completely revolutionized birth control by offering a fail-safe way to prevent pregnancy. The Norplant family of contraceptive delivery devices is fabricated from Silastic, a silicone elastomer. However, with Silastic side effects are not uncommon. The most common clinical finding among Norplant users was a local inflammatory response to the implant, which resolves on removal of the implant. However, the controversies surrounding the silicone breast implants have fueled a debate that brings into question the safety of synthetic polymers in medicine and medical devices. Typical sources for the induction of adverse tissue response to an implant are species that leach out of the polymer matrix. These species are termed as leachants. They include unreacted or residual monomer; residual catalyst and their degradation products; low-MW polymers; fillers; and sterilization agents, such as ethylene oxide.

In recent years, a nondegradable polymer, polyethylene-co-vinyl acetate (pEVA) has been explored for application in various medical products worldwide. In the case of an ethylene vinyl acetate co-polymer (EVA) system, the most likely leachants are unreacted acetic acid ethyl ester (vinyl acetate monomer or VAM). Several studies looking at the carcinogenicity of VAM in rats and mice have shown VAM to cause an increase in total malignant tumors and in carcinomas and/or precursor lesions of the oral cavity, lips, tongue, esophagus, and forestomach. Based on these data, VAM must be considered a multipotent carcinogen.¹ It has also been shown to be a teratogen to human cells in vivo. The International Agency for Research on Cancer (IARC) classified vinyl acetate as a possible carcinogenic in humans.² EVA is available from numerous sources with varying degrees of VAM present. The present invention relates to the selection of EVA with low VAM content below 0.5% W/W or EVA
¹Minardi F et al. Results of Long-Term Carcinogenicity Bioassay on Vinyl Acetate Monomer in Sprague-Dawley Rats. Annals of the New York Academy of SciencesVolume 982, Issue 1, 2006.
²NTP-Working-Group. Toxicology and carcinogenesis studies of polyvinyl alcohol (molecular weight approx. 24,000) in female B6C3F1 mice (intravaginal studies). National Toxicology Program Technical Reports Series PG. 1998;474:109.
with non-detectable levels of VAM at or even below the limit of quantification (LOQ).

In the laboratories of the applicant, also levonorgestrel (LNG)-releasing EVA-based fibers have been developed for contraception and the treatment of gynecological conditions such as the treatment of heavy menstrual bleeding (menorrhagia). These fibrous delivery systems typically release 10 to 20 µg of LNG/d and have maximal efficacy for 5 years. Abnormal uterine bleeding is the most frequent gynecological complaint and the incidence increases as the woman approaches menopause. Half of these women have a hysterectomy within 5 years if conservative treatment (e.g. contraceptive pills, progestogens, fibrinolytic inhibitors and prostaglandin inhibitors) fails.

Uterine fibroids (fibromyoma, leiomyoma) are also a prevalent condition, with a cumulative incidence by age 50 of nearly 70% in white women and greater than 80% in black women. Uterine fibroids result in an annual societal cost in the United States, that surpasses that of breast cancer, colon cancer and ovarian cancer combined, and nearly one fifth the annual cost of diabetes in the United States.

Endometriosis is yet another frequent, debilitating and costly condition in women characterized by the presence of endometrial tissue outside the uterus. Endometriosis typically occurs on the surface of organs in the abdomino-pelvic cavity and recto-vaginal septum and is a chronic painful condition. Endometriosis affects almost 10-20% of women of reproductive age, while 70-90% of women with chronic pelvic pain (CPP) also have endometriosis, a disease that impairs patients' quality of life.

Adenomyosis, which is characterized by the presence of ectopic endometrial glands in the uterine wall, associated with hyperplasia of the surrounding muscles, is present in at least 1% of the female population. The symptoms are more or less similar as in endometriosis. Heavy menstrual bleeding is often associated with adenomyosis.

A number of studies have estimated the prevalence of chronic pelvic pain (CPP) to be similar to that reported for migraine, low back pain and asthma. Dysmenorrhea (painful menstruation) and endometriosis are the two most common causes of pelvic pain.

Hysterectomy is the most commonly used procedure to treat heavy menstrual bleeding and fibroids although medical treatment are preferable. Mild cases of endometriosis are managed medically with contraceptive steroids and non-steroidal anti-inflammatory agents. Surgery provides relief to women in pain but symptoms recur in 75% of cases within 2 years.

Because of the serious potential side effects caused by the use of systemically administered medications, as well as compliance issues, new, long-acting, therapeutic options are currently under investigation. One of these options is the levonorgestrel-releasing intrauterine system (LNG-IUS). This system has been tested with success in women with heavy menstrual bleeding, endometriosis and adenomyosis. Fibromyomas are often accompanied by heavy menstrual bleeding which can be treated effectively by the LNG-IUS. The LNG-IUS is also highly effective contraceptive. However, higher dosages than those used for contraception may be necessary for the treatment of the gynecological pathologies described above. The way how this can be achieved is addressed in the present application.

Orally administered progesterone antagonists (PA) and selective progesterone receptor modulators (PRMs) have been highly satisfactory for the conservative treatment of fibroids or for reducing the fibroid size prior to surgery. The fibroids can be reduced is size by up to 50 % or more. Clinical results with orally administered PA and PRMs are, therefore, promising and superior to other treatment modalities. However, when the treatment is interrupted recurrence rates are high indicating the need for long-term treatment. In addition, adverse effects of long-term systemically administered PA and PRMs have been observed on the endometrial level and in liver function. Endometrial thickening has been found in many patients using these compounds and have been associated with cystic dilatation and hyperplasia. The endometrial effect of systemically applied PA and PRMs results in breakthrough bleeding and even in heavy bleeding. Of greater concern is the abnormalities in liver function which are usually minor. However, they resulted in the withdrawal of one PRM from clinical trials.

Local delivery, in the uterus or vagina, of PA/PRM may act more profoundly than orally administered PA/PRM as has been demonstrated with LNG. Local therapy may be used in conjunction with oral treatment for long-term suppression. A long-acting delivery system, in addition to providing optimal patient compliance and eliminate effects on ovarian function and other potential systemic side effects may, therefore, be preferable to systemic treatment schedules. Local therapy using PA/PRM has not been used to control fibroids and endometriosis. Intrauterine treatment might target myoma and endometriosis specific symptoms better and be more effective in reducing pelvic pain and pressure due to the size of the tumor, and abnormal uterine bleeding. It was proposed that local delivery of progesterone antagonistic drugs, with its strong anti-progestogen activity and anti-estrogenic effect at the endometrial level, are likely to be more suitable than LNG to reduce the size of the fibroid tumors and to have an effect on endometriotic tissue. No studies with intrauterine delivery of progesterone antagonists and PRMs have been conducted so far, except in primates. These studies showed that progesterone antagonists and PRMs suppress the endometrium, induce amenorrhea and inhibit fertility. PA/PRM-releasing IUSs may therefore provide an effective treatment modality that also controls endometrial bleeding. PA/PRM-IUSs suppress the effects of progesterone on endometrial progestational development and block the effects of estrogen on endometrial proliferation.

As the lesions described above are often located deep in the tissues of the genital tract, the present invention describes ways how to increase the release rate, to allow the PA/PRM to penetrate in the deeper layers of the genitalia, using an intrauterine system of small dimensions, that is inserted in the uterine cavity.

An important aspect in relation to intrauterine drug delivery for contraception and treatment is the size and shape of the uterine cavity that differs from woman to woman, being parous or nulliparous. Nulliparous women are particularly vulnerable to geometric incompatibility. If the IUS is too big for the uterine cavity, pain complaint will occur and the uterus may expel the foreign body. Furthermore, uterine cavities are often deformed or distorted due to the presence of multiple fibromyomas in the wall of the uterus. In this case a "framed" IUS may be less suitable than a "frameless" one.

The frameless characteristics of an "anchored" IUS were first discussed in 1986 (European Patent EP0191747), and further improved in published patent application WO 2011/080164. In this application the use of different strands of co-axial fibers were described.

Similarily, in 2003, and later in 2010, International Patent Publication W02003/0068117 and W02010/070150 respectively described improved intrauterine "framed" devices. The retention mechanism of these IUSs consists of plastic retention arm which take position against the lateral walls of the uterus after insertion to prevent expulsion of the IUS. In order to maximize the release rate of the PA/PRM, the plastic retention arm could be replaced by a fibrous delivery rods or fiber, with sufficient rigidity to act as retention means in the uterus. Both monolithic (first-order) and bicomponent (zero-order) fibers could be considered. Alternatively, the plastic tail of the IUS could be replaced by a drug-releasing fiber or a drug releasing fiber could be added to the bottom of the IUS.

The purpose of the present invention is to provide several kinds of drug delivery systems consisting of at least two drug delivery systems, compatible with the geometric dimensions of the uterine cavity, releasing one or more bioactive substances, such as steroids in combination with other agents, acting as a contraceptive, and that are simultaneously also able to prevent or treat a gynecological disorder, endocrine disorder or disorders in the genital, pelvic and reproductive tract (e.g., uterus or cervix) of women, or prevent ascending infection, and of which the release characteristics are such that at least one of these systems can release the drug fast during the initial period after its insertion and of which the release characteristics of the other drug delivery system is active over a long period of time, ideally five years, but in various instances and in various formulation up to ten or fifteen years, and of which the drug delivery systems consist of ethylene vinyl acetate, functioning as reservoir for a drug substance, contains virtually undetectable levels of the carcinogenic vinyl acetate monomer to guarantee the safety of the long-lasting drug eluting system.

### SUMMARY OF THE INVENTION

The present invention relates to a multipurpose bioactive agent-releasing intrauterine system for use in women with an ideal lifespan of between 1 month to five years but may be capable for use of up to ten years or more, consisting of an uni- or multisegmented stem of reduced dimension, functioning as drug delivery compartment(s), of which the upper and lower extremities are dome-shaped, to allow easy insertion in the uterine cavity with a thin inserter tube, and of which the stem consists of a particulate blend of a hormone or bioactive substance(s) dispersed in an excipient which consists of ethylene vinyl acetate and contains sufficient hormone, released in such small quantities per day, by using a rate controlling membrane with such specific characteristics that both fast and sustained delivery for a minimum of 0.1 years, ideally up to five and up to more than ten years; and is provided further with a retention arm, bioactive or not, or an uterine anchor, to retain the system in the uterus.

Furthermore the drug delivery compartment can be used in various conditions where long-term constant administration of the bioactive agent would be clinically beneficial. The regional/local delivery of bioactive substances via these drug delivery system(s) will serve to enhance effectiveness, reduce adverse events, improve compliance and ultimately benefit to the patient.

This purpose is reached according to the present invention by providing a drug delivery system comprising:
at least one metabolically active agent, dispersed in an excipient, said device being arranged to form a non-rigid structure, **characterized in that** it consists of a core which consists of ethylene vinyl acetate or other suitable polymer or polymer blend, the excipient, and an active substance useful in gynecological, endocrine or other relevant disorders, and a rate controlling membrane. This fiber or rod, or two or more fibers or rods in succession, could be used to form one component of an IUS, such as the stem or retention arm of the device, or be used as a ring-shaped device for intravaginal drug release or as a rod-shaped subdermal or intraorgan implant/insertion.

According to one aspect of the present invention, the drug delivery rod is used as the stem of an intrauterine system.

According to another aspect of the invention, the drug delivery rod is used not only to constitute the stem of the intrauterine system but also the retention arm.

According to another aspect of the invention, the drug delivery system is a fiber, or a succession of segments of fibers, attached to an anchoring means to suspend the fiber to the wall of the uterus or other biological cavity.

According to another aspect of the invention, the drug delivery system is a fiber, or a succession of segments of fibers, attached to an anchoring means, of which the release rate can be adapted to the individual need of the woman by providing a means to trim the length of the fiber or allowing one or more segments of the fiber to be removed.

According to another aspect of the invention, the anchor is a specially designed knot, described in detail below, to maximize its retention in the tissue of the uterus.

According to an additional aspect of the invention, the drug delivery compartment is combined with copper/silver/gold elements, attached to the intrauterine system to provide contraception and possible infection prevention.

According to yet another aspect of the present invention, the drug delivery rod or fiber or tail of the IUS contains, or is covered with a polymer sleeve loaded with macro, micro or nanoparticle form of the bioactive substance combined with an appropriate polymer or polymer blends (e.g., EVA) for long term use in the body of men, women and other mammals.

According to another aspect of the present invention, the drug delivery rod or fiber or tail of the IUS contains, or is covered with a polymer sleeve loaded with macro, micro or nanoparticle form of the bioactive substance combined with an appropriate polymer(e.g., EVA, PET) and a rate controlling membrane of EVA or other appropriate polymer(s) alone or as a blend for long term use in the body.

According to another aspect of the present invention, the drug delivery rod or fiber or tail of the IUS contains, or is covered with a polymer sleeve loaded with micro or nanoparticle form of LNG combined with an appropriate polymer(e.g., EVA) and a rate controlling membrane of EVA or other appropriate polymer(s) alone or as a blend for long term use in the body as an intrauterine contraceptive.

According to another aspect of the present invention, the drug delivery rod or fiber provides a regional or local release of the bioactive substance at a lower daily rate at reduced dose seen systemically to treat a condition with a reduction in systemic exposure to the bioactive substance and side effects.

According to another aspect of the present invention, the drug delivery rod or fiber or tail of the IUS contains, or is covered with a polymer sleeve loaded with macro, micro or nanoparticle form of the bioactive substance combined with an appropriate polymer(s)(e.g., EVA) alone or as a mixture for long term use in the body utilizing a dual extrusion system.

According to another aspect of the present invention, the drug delivery rod or fiber or tail of the IUS contains, or is covered with a polymer sleeve loaded with micro or nanoparticle form of the bioactive combined with an appropriate polymer(s)(e.g., EVA) alone or as a mixture and a rate controlling membrane of EVA or other appropriate polymer(s) alone or as a blend for long term use in the body using a bioactive agent alone or in combination selected form the class of estrogens, GnRH agonist, GnRH antagonist, progestins, anti-progestins, anti-estrogens, aromatase inhibitors, androgens, glucocortoid, anti-inflammatory and chemotherapeutic agents, thrombolytics, vasodilators, microbicides, antivirals, vaccines, peptides, peptide mimics.

According to a further aspect of the present invention, the drug delivery rod contains EVA with VAM content below 0.5% or even at or below the limit of quantification in the core as well as the rate controlling membrane for long term use in the body of men, women and other mammals.

According to another aspect of the present invention, the drug delivery rod is used as the stem of an intrauterine system, of which both extremities are dome-shaped with T-shaped retention arm suitable for effective contraception of five to ten year duration without replacement.

According to a further aspect of the invention, the dome consists of an inner part which is the polymer-hormone blend, and the outer membrane that covers the inner part completely or partially, at the apex of the dome.

According to a another aspect of the invention, the dome consists of denser material and serves to make the extremities of the drug delivery rod visible on ultrasound.

According to yet another aspect of the invention, the aforementioned construction of the drug delivery rod can be used as a subdermal or intraorgan implant. The dome-shaped extremities will then serve to avoid damage or irritation to the surrounded tissue.

According to another aspect of the invention, the fiber extremities are provided with a small metal element inserted in the core of both fiber extremities to allow visualization of the drug delivery rod by ultrasound or X-ray when inserted subdermally.

According to another aspect of the invention, the drug delivery rod is folded to construct a ring-shape of which both extremities are welded together to close the ring.

According to another characteristic of the invention, the vaginal ring consists of different segments which have an identical or different composition, monolithic or coaxial, and of which the active substance released from each of the segments has the same or a different molecular structure.

According to yet another aspect of the invention, the drug delivery rod is between one and five millimeter in diameter in the case the rod is used as the stem and/or the retention arm of an intrauterine system and between one and five millimeter in case a vaginal ring is constructed.

According to an additional aspect of the invention, the mean release profile of the fibrous delivery system follows zero-order or apparent zero-order release characteristics for up to approximately the first five +/- one years followed by a complex mixed zero/first-order release for an additional period of up to five +/- one years, or vice versa.

According to another characteristic of the invention, the initial release rate during the first month up to three months after implantation is eight to ten times higher than the mean release rate over the next five year period.

According to an additional embodiment of the invention, the rate controlling membrane, encapsulating the core of the drug delivery compartment is between 0.1 and 0.4 millimeter in the mid-section of the body of the drug delivery rod, and between 0.3 and 0.6 millimeter at both dome-shaped extremities of the compartment in the case an intrauterine system or subdermal/intraorgan implant is constructed.

According to yet another embodiment of the invention, the dimension of the rate controlling membrane can be double or triple that compared to thickness of the drug delivery membrane of an intrauterine drug release system or subdermal /intraorgan implant in case an intravaginal ring is constructed, depending on the drug compound.

According to another embodiment of the invention, the length of the drug delivery (multisegmented) compartment is between two and twelve centimeter in the case of an intrauterine system, both the stem and retention arm combined, as well as in the case of one of two subdermal implants.

According to another embodiment of the invention, the ring-shaped drug delivery system has a diameter between five and seven centimeter.

According to a preferred mode of realization, the fibrous drug delivery system consist of a drug delivery compartment that is three to four centimeter long and between 1.6 and 2.5 millimeter in diameter, of which the outer sheet or membrane is between 0.05 and 0.4 millimeter in thickness; and which is provided with dome-shaped extremities, closed at both ends by the outer sheet which covers the inner core partially or totally at the apex of the dome; and of which both the core and membrane consist of ethylene vinyl acetate, with wt% between 24% and 29%, and which contains low levels of vinyl acetate monomer (< LOQ); and of which the reservoir or multisegmented reservoirs are loaded with bioactive substance(s) singularily or in combination (e.g.,the class of estrogens, GnRH agonist, GnRH antagonist, progestins, anti-progestins , anti-estrogens, aromatase inhibitors, androgens, glucocortoid, anti-inflammatory and chemotherapeutic agents, thrombolytics, vasodilators, microbicides, antivirals, vaccines, peptides, peptide mimics).

### DESCRIPTION OF THE DRAWINGS

These and other characteristics of the invention will be more readily understood when referring to the description as well as the accompanying drawings which represent, merely by way of non-limitative examples, several embodiments of the invention, and in which:
FIG. 1 represents a view of a preferred embodiment of the drug delivery system according to the invention of which the drug delivery rod constitutes the "segmented", "multipurpose" stem of a T-shaped intrauterine system.
FIG. 2 represents another view of a preferred mode of realization according to the invention consisting of a drug delivery rod serving as stem and a second drug delivery rod which constitutes the retention arm of a T-shaped intrauterine system.
FIG. 3 represents still another view of a preferred embodiment of the drug delivery system according to the invention of which the segmented, multipurpose drug delivery fiber(s) is the active component of a frameless, anchored intrauterine system or which consists of two different drug delivery systems.
FIG. 4 represents another view of a preferred mode of realization according to the invention of which the plastic retention arm and/or the tail of the device is provided with copper and or silver or gold tubes for contraceptive and anti-infectious purposes.
FIG. 5 represents another view of a preferred mode of realization according to the invention of which the tail of the T-shaped device or a second strand consists of a drug delivery system releasing a bioactive substance.
FIG 6. represents another view of a preferred mode of realization according to the invention of which the tail of the anchored, frameless device or a second or third strand consists of a drug delivery system releasing a bioactive substance.
FIG. 7. is another realization according to the invention in which a polymeric sleeve loaded with bioactive nanoparticles affixed to the tail of the intrauterine device.
FIG. 8. is yet another realization according to the invention in which a polymeric sleeve loaded with bioactive nanoparticles covers partly the stem of the intrauterine device.
FIG. 9 represents another view of a preferred mode of realization according to the invention of which the extremities of the drug delivery rod are dome-shaped of which the apexes of the dome can be open or closed.
FIG. 10 represents another view of a preferred mode of realization according to the invention of which the extremities of the drug delivery rod are dome-shaped and of which the center of the dome contains a small metal element to allow visualization on ultrasound and X-ray.
FIG. 11 represents another view of a preferred mode of realization according to the invention of which the drug delivery rod is folded to form a ring-shaped drug delivery device for intravaginal drug delivery.
FIG. 12 represents a schematic representation of a multisegmented vaginal ring consisting of monolithic and coaxial fibers, or co-axial fibers with different physical and release characteristics, segments welded together to form the ring.
FIG. 13 represents how the anchoring knot is made.
FIG. 14 is a photograph of the preferred mode of realization of the anchoring knot.

### DETAILED DESCRIPTION

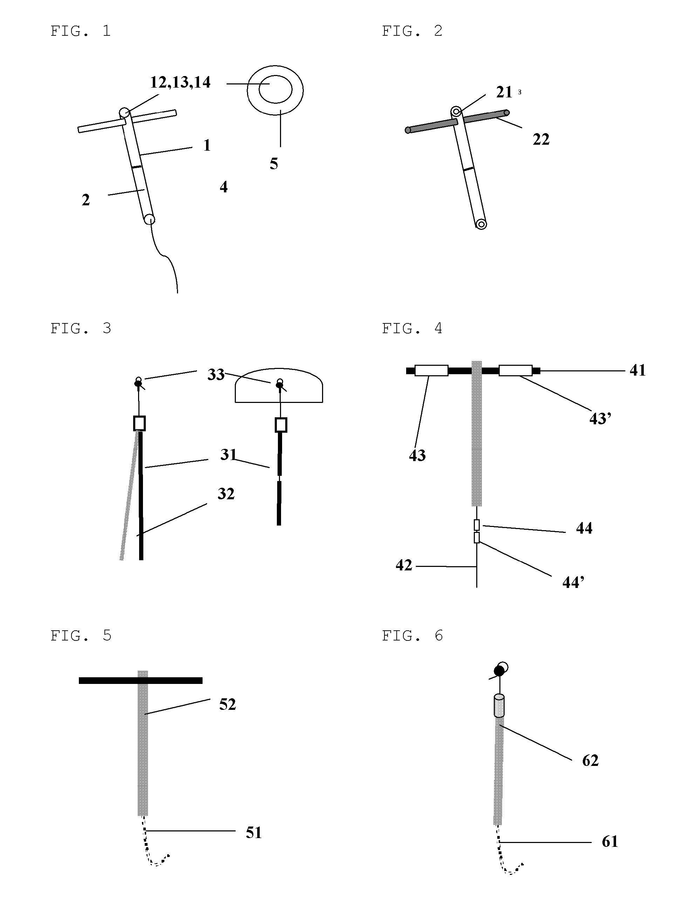

Referring now particularly to FIG. 1, a drug delivery system 11, multi-segmented, with a different chemical composition and release characteristics, or not, constituting the stem of a T-shaped intrauterine system with horizontal retention arm 12, of which the stem is a coaxial rod with core consisting of ethylene vinyl acetate 13, loaded with a drug 14; and a rate controlling membrane 15; and of which the retention arm 12 is made of plastic.

Referring to FIG. 2, a drug delivery system 21 constituting the stem of a T-shaped intrauterine system with horizontal retention arm 12 which is also a drug delivery system.

Referring to the embodiment of the representation in FIG. 3, the fibrous drug delivery systems 31 and 32 are different in composition and release characteristics, and or multisegmented, acting as the active component of a "frameless" intrauterine system with anchor 33 to retain the system in the uterus.

As shown in FIG. 4 which refers to Fig. 1, the plastic retention arm 41 and the tail 42 of the device are provided with copper, silver or/gold tubes , or a combination, 43 and 43' and 44 and 44' to provide contraception and possible infection prevention.

As shown in FIG. 5 the tail 51 of the T-shaped device 52 consists of a drug delivery system releasing a bioactive substance.

As shown in FIG 6 the tail 61 of the frameless device 62 consists of a drug delivery system releasing a bioactive substance.

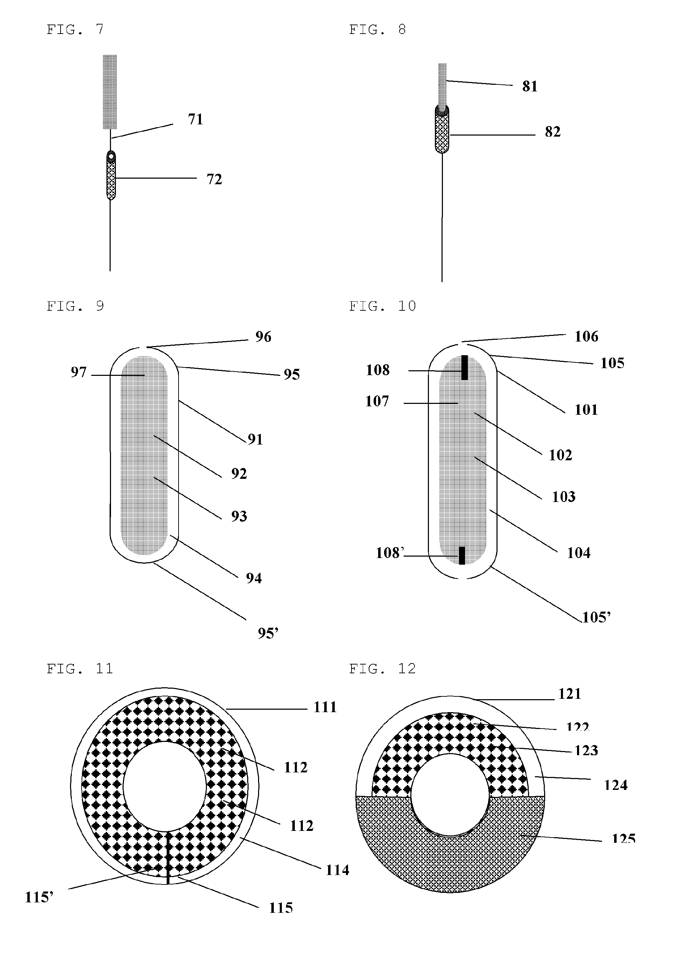

FIG. 7 is a detail of one component of the device, the tail 71 of the IUD, which is covered by a soft polymer tube 72 loaded with metallic nanoparticles for the prevention of infection.

FIG. 8 is a detail of another component of the device, the stem 81 of the IUD, which is partly covered by a soft polymer tube 82 loaded with metallic nanoparticles for the prevention of infection.

FIG. 9 is another schematic sectional view of a coaxial fiber or rod 91 loaded with a bioactive substance 72 dispersed in an excipient 93; and having a rate controlling membrane 94 of which the proximal and distal ends, 75 and 95' are dome shaped, the apex 96 of the dome being open or closed covering the core 97 of the rod.

FIG. 10 is another schematic sectional view of a coaxial fiber or rod 101 loaded with a bioactive substance 102 dispersed in an excipient 103; and having a rate controlling membrane 104 of which the proximal and distal ends, 85 and 85' are dome-shaped, the apex 86 of which being open or closed covering the core 107 of the rod and of which the dome contains a metal element 108 and 108' in its center to allow visibility on ultrasound and X-ray.

FIG. 11 is another schematic sectional view of a coaxial fiber 111 loaded with a bioactive substance 112 dispersed in an excipient 123; and having a rate controlling membrane 114 of which the fiber is folded to form a ring of which both the proximal and distal ends, 115 and 115' are welded to close the ring.

FIG. 12 is a schematic view of a multi-segmented vaginal ring 121, loaded with a bioactive substance 122 dispersed in an excipient 123; and having a rate controlling membrane 124 in one segment of the ring and is monolithic, or co-axial with a different chemical composition of the membrane in another segment 125 of the ring, both welded together to close the ring.

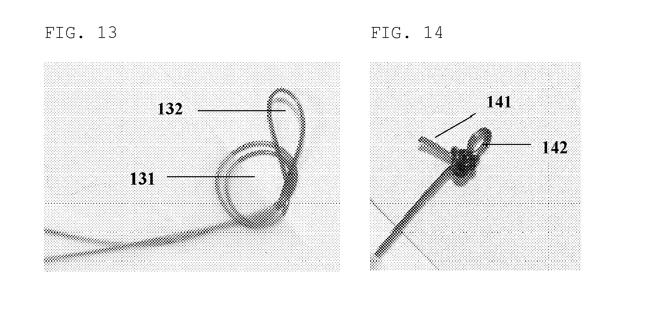

FIG. 13 is a representation how the anchoring knot is made to make it consistent and uniform. After the first loop 131 is made, a second loop 132 is put through the first loop and tied on a fixture.

FIG. 14 is a photograph showing the anchoring knot in detail. The short end of the knot 141 helps retain the knot in the tissue. The noose 142 adapts to the tip of a specially designed stylet to allow insertion of the knot in the uterine tissue.

The invention is therefore directed among others to a device or system having the following features, separately or in any combination :
- it is a multipurpose, multicomponent or multisegmented intrauterine T-shaped drug delivery device, of which both the stem and the transverse arm of the device are drug eluting compartments, that have different properties simultaneously, contraceptive, therapeutic and preventive, as well as anti-infectious properties, incorporating the same or different active pharmaceutical ingredients, and using the same or different polymeric systems, that constitute the device, and which is active in the uterus, as well as beyond the uterus, and which can be adapted to the transverse dimensions of the uterine cavity, and of which the stem of the device consists of one or more drug delivery compartments, possibly extending into the cervical canal, with length between two to eight centimeter and between 1.0 and 3.0 millimeter in diameter, of which the outer sheet or membrane is between 0.05 and 0.4 millimeter in thickness; and which is provided with dome-shaped extremities, closed at both ends by the outer sheet which covers the inner core partially or totally at the apex of the dome; and of which both the core and membrane consist of ethylene vinyl acetate, with wt% between 8% and 35%, (ideally between 24% and 29%) and which contains low levels of vinyl acetate monomer (<0.5% w/w)),
- the dual or multisegmented drug delivery compartment(s), being the active component(s) of the intrauterine system is attached to a frameless suspension system provided with an anchoring means to retain the system in the uterus,
- it consists of a core of ethylene vinyl acetate, loaded with a bioactive substance and rate controlling membrane consisting of EVA, using EVA with VAM content of <0.5% (w/w) for use as an intrauterine contraceptive device in women, and of which the tail(s) consists also of a drug delivery system(s),

- the fibrous drug delivery compartment attached to the frameless drug delivery system, and the tail, loaded with an active component, can be trimmed or removed to adapt to the uterine dimensions and/or to reduce the release rate,
- there is a retention means consisting of a suture knot which is specially designed to be consistent and uniform and can function as a biocompatible anchor when inserted in the wall of the uterus,
- the total loading of drug in the intrauterine system is between 30 and 150 mg or more,
- the drug releasing rate is between 5 and 150 µg/d or more,
- it consists of two identical or different drug delivery systems, one constituting the stem of an intrauterine system and the other the transverse arm or retaining member.
- both proximal and distal ends are dome-shaped, a membrane covering the inner core completely or partially, with ends or extremities having a more dense structure, allowing greater visibility on ultrasound.
- the extremities of the drug delivery rod contain a metal element rendering the implant visible on X-ray, and which are kept in place by the denser structure of the dome-shaped fiber extremity.
- the excipient is ethylene vinyl acetate which has undergone a purification process to reduce (<0.5% w/w) or eliminate vinyl acetate monomer to a level below the limit of quantification.
- the loading of the core with bioactive substance is at least 10% up to more than 50%, evenly dispersed in the excipient.
- the rate controlling membrane, encapsulating the core of the drug delivery compartment, is between 0.1 and 0.4 millimeter in the mid-section of the body of the drug delivery rod, and between 0.3 and 0.6 millimeter at both dome-shaped extremities of the rod/fiber in the case an intrauterine system or subdermal/intraorgan implant or insertion device into body cavities is constructed.
- the mean release profile of the fibrous delivery systems follows a complex mixed zero/first-order release for an additional period of one year or more.
- the initial release rate during the first month up to three months after implantation is eight to ten times higher than the mean release rate over the next three to five year period.
- the drug delivery rod is between one and five millimeter in diameter in the case the rod is used as the stem and/or the transverse/retaining arm of an intrauterine system or as a subdermal intraorgan implant or insertion device into body cavities.
- at least one drug delivery compartment is combined with copper, silver or gold elements, on the transverse arm and/or on the tail of the device, to provide contraception and possible infection prevention.
- at least one of the drug delivery rods contains micro or nanoparticle form of the bioactive substance combined with an appropriate polymer (e.g., EVA) for long term use.
- the tail is coated with micro or nanoparticle form of a bioactive substance such as silver, gold, magnesium oxide, copper oxide or a combination for release in the cervical canal.
- the active ingredient(s) is chosen endogenous hormones, estrogens, progestogens, antiprogestogens, selective progesterone modulators, androgens, GnRH agonist, GnRH antagonist, anti-estrogens, aromatase inhibitors, glucocortoid, anti-inflammatory and chemotherapeutic agents, thrombolytics, vasodilators, microbicides, antivirals, vaccines, peptides.
- it consists of a core of ethylene vinyl acetate, loaded with a bioactive substance and rate controlling membrane consisting of EVA with VAM content of <0.5% (w/w) for use as an intrauterine contraceptive device in women.

Also disclosed is a subdermal contraceptive device in women.

Also disclosed is a novel multisegmented ring-shaped device which consist of a mixture of different monolithic or coaxial fibrous delivery systems, or a mixture of both, releasing the same or different drugs at a different rate.

Also disclosed is a anchoring knot having a noose of which the inner diameter is between 0.6 and 1.0 mm, to suspend bioactive substances in the uterine cavity, consisting of biocompatible material constructed such that it can easily be inserted in the tissue of the uterus, using a specially designed stylet.

A monofilament anchoring means with a specific design consisting of a knotted part with noose and appendix is also disclosed.

## Claims

1. A multipurpose, multicomponent or multisegmented drug intrauterine T-shaped delivery system or device, of which both the stem and the transverse arm of the device are drug eluting compartments which are active in the uterus and/or the cervical canal.

2. An intrauterine delivery device according to claim 1 wherein the drugs are two different drugs.

3. An intrauterine delivery device according to claim 1 wherein the drugs are the same but have different concentrations or loading, as well as different release characteristics, in said compartments.

4. An intrauterine delivery device according to any preceding claims wherein the stem is provided with at least one dome-shaped extremity.

5. An intrauterine delivery device according to any preceding claims wherein the stem is itself multisegmented to provide different compartments.

6. An intrauterine delivery device according to any preceding claims wherein the stem and/or the transverse arm consist of ethylene vinyl acetate, with wt% between 8% and 35%, preferably between 24% and 29%, and which contains a level of vinyl acetate monomer at <0.5% w/w.

7. An intrauterine delivery device according to any preceding claims wherein the stem is extending into the cervical canal.

8. An intrauterine delivery device according to any preceding claims wherein the length of the stem is between 2 to 8 cm.

9. An intrauterine delivery device according to any preceding claims wherein the diameter of the stem is between 1.0 to 3.0 mm.

10. An intrauterine delivery device according to any preceding claims wherein the stem and/or transverse arm consist of a core and an outer membrane.

11. An intrauterine delivery device according to any preceding claims wherein the membrane covers at least one extremities partially or totally the apex of the stem and/or transverse arm.

12. An intrauterine delivery device according to any preceding claims wherein the membrane is between 0.05 and 0.4 mm in thickness.

13. A drug delivery device or system, according to any preceding claims, wherein one of the dual or multisegmented drug delivery compartments, preferably the stem at its upper portion, is attached to a frameless suspension system provided with an anchoring means in the fundus of the uterine in order to retain the system in the uterus.

14. A drug delivery device or system, according to any preceding claims which is provided with a tail, preferably a thread, attached to the lower end of the stem.

15. A drug delivery system, according to the preceding claim wherein the tail consists also of a drug delivery compartment, preferably an antimicrobial drug compartment.
